# EUROPEAN PATENT APPLICATION

(11) **EP 4 806 914 A1**
(43) Date of publication of application: **16.09.2026**
(21) Application number: 24888712.7
(22) Date of filing: 05.11.2024
(51) Int. Cl.: C07K 16/08, C07K 1/22, C07K 17/00, C12N 1/15, C12N 1/19, C12N 1/21, C12N 5/10, C12N 15/13, C12N 15/864, C12P 21/08

(54) **AFFINITY POLYPEPTIDE FOR FULL CAPSIDS OF AAV**

(30) Priority: 06.11.2023 JP 2023189677
(71) Applicant: Daicel Corporation, Osaka-shi, Osaka 530-0011 (JP); NATIONAL UNIVERSITY CORPORATION KYOTO INSTITUTE OF TECHNOLOGY, Kyoto-shi, Kyoto 606-8585 (JP)
(72) Inventor: OKURA, Hiromichi, Tokyo 108-8230 (JP); HIRABAYASHI, Yuki, Tokyo 108-8230 (JP); KUMADA, Yoichi, Kyoto-shi, Kyoto 606-8585 (JP)
(74) Representative: Grünecker Patent- und Rechtsanwälte PartG mbB
(86) International application number: PCT/JP2024/039335
(87) International publication number: WO 2025/100417

(57) **Abstract**

An object of the present disclosure is to provide an affinity molecule having higher binding affinity for AAV full capsids than for AAV empty capsids. An anti-AAV full capsid molecule, which includes a heavy chain variable region including, as heavy chain complementarity-determining regions, CDR1 set forth in SEQ ID NO: 6, CDR2 set forth in SEQ ID NO: 7, and CDR3 set forth in SEQ ID NO: 8; and a light chain variable region including, as light chain complementarity-determining regions, CDR1 set forth in SEQ ID NO: 9, CDR2 consisting of RAS, and CDR3 set forth in SEQ ID NO: 10, has higher binding affinity for AAV full capsids than for AAV empty capsids.

## Description

### Technical Field

The present disclosure relates to an affinity polypeptide for full capsids of adeno-associated virus (AAV).

### Background Art

In the production process of an adeno-associated virus (AAV) applied as a gene therapy drug, a mixture of full capsids, which encapsulate foreign DNA within the virus to serve as an active pharmaceutical ingredient, and empty capsids, which contain no encapsulated material, is obtained. In the production process, a method for quantifying full capsids from the mixture is required.

As a specific method for quantifying full capsids, quantitative PCR (Patent Document 1), interferometric scattering mass spectrometry (Patent Document 2) are known.

### Citation List

### Patent Document

Patent Document 1: CN 101426935 B
Patent Document 2: WO 2021/191079

### Summary of Invention

### Technical Problem

Methods for quantifying full capsids using quantitative PCR or interferometric scattering mass spectrometry require expensive dedicated equipment, and lack simplicity and versatility.

In order to quantify full capsids by a simple and versatile method, use of an affinity molecule is desired. An affinity molecule suitable for such quantification of full capsids is required to have a higher binding affinity for AAV full capsids than for AAV empty capsids (i.e. selective binding to AAV full capsids).

Accordingly, an object of the present disclosure is to provide an affinity molecule having higher binding affinity for AAV full capsids than for AAV empty capsids.

### Solution to Problem

As a result of intensive studies, the present inventor has found an anti-AAV full capsid molecule having higher binding affinity for AAV full capsids than for AAV empty capsids. The present disclosure is completed through further examinations based on this finding.

That is, the present disclosure provides the invention as set forth in the following aspects.
Aspect 1: An anti-AAV full capsid molecule that is an antibody having higher binding affinity for AAV full capsids than for AAV empty capsids.
Aspect 2: An anti-AAV full capsid molecule including:
   a heavy chain variable region including, as heavy chain complementarity-determining regions, CDR1 consisting of an amino acid sequence set forth in SEQ ID NO: 1, CDR2 consisting of an amino acid sequence set forth in SEQ ID NO: 2, and CDR3 consisting of an amino acid sequence having 90% or more sequence identity with an amino acid sequence set forth in SEQ ID NO: 3; and
   a light chain variable region including, as light chain complementarity-determining regions, CDR1 consisting of an amino acid sequence set forth in SEQ ID NO: 4, CDR2 consisting of RAS, and CDR3 consisting of an amino acid sequence having 90% or more sequence identity with an amino acid sequence set forth in SEQ ID NO: 5.
Aspect 3: An anti-AAV full capsid molecule including:
   a heavy chain variable region including, as heavy chain complementarity-determining regions, CDR1 consisting of an amino acid sequence set forth in SEQ ID NO: 6, CDR2 consisting of an amino acid sequence set forth in SEQ ID NO: 7, and CDR3 consisting of an amino acid sequence having 90% or more sequence identity with an amino acid sequence set forth in SEQ ID NO: 8; and
   a light chain variable region including, as light chain complementarity-determining regions, CDR1 consisting of an amino acid sequence set forth in SEQ ID NO: 9, CDR2 consisting of RAS, and CDR3 consisting of an amino acid sequence having 90% or more sequence identity with an amino acid sequence set forth in SEQ ID NO: 10.
Aspect 4: An anti-AAV full capsid molecule including:
   a heavy chain variable region including, as heavy chain complementarity-determining regions, CDR1 consisting of an amino acid sequence set forth in SEQ ID NO: 11, CDR2 consisting of an amino acid sequence set forth in SEQ ID NO: 12, and CDR3 consisting of an amino acid sequence having 90% or more sequence identity with an amino acid sequence set forth in SEQ ID NO: 13; and
   a light chain variable region including, as light chain complementarity-determining regions, CDR1 consisting of an amino acid sequence set forth in SEQ ID NO: 14, CDR2 consisting of QAS, and CDR3 consisting of an amino acid sequence having 90% or more sequence identity with an amino acid sequence set forth in SEQ ID NO: 15.
Aspect 5: An anti-AAV full capsid molecule including:
   a heavy chain variable region including, as heavy chain complementarity-determining regions, CDR1 consisting of an amino acid sequence set forth in SEQ ID NO: 16, CDR2 consisting of an amino acid sequence set forth in SEQ ID NO: 17, and CDR3 consisting of an amino acid sequence having 90% or more sequence identity with an amino acid sequence set forth in SEQ ID NO: 18; and
   a light chain variable region including, as light chain complementarity-determining regions, CDR1 consisting of an amino acid sequence set forth in SEQ ID NO: 19, CDR2 consisting of RAS, and CDR3 consisting of an amino acid sequence set forth in SEQ ID NO: 20.
Aspect 6: The anti-AAV full capsid molecule according to any one of Aspects 2 to 5, wherein
   the heavy chain variable region includes:
      FR1 consisting of an amino acid sequence having 70% or more sequence identity with an amino acid sequence at positions 1 to 24 of SEQ ID NO: 21, positions 1 to 24 of SEQ ID NO: 23, positions 1 to 24 of SEQ ID NO: 25, or positions 1 to 24 of SEQ ID NO: 27;
      FR2 consisting of an amino acid sequence having 70% or more sequence identity with an amino acid sequence at positions 33 to 49 of SEQ ID NO: 21, positions 34 to 50 of SEQ ID NO: 23, positions 34 to 50 of SEQ ID NO: 25, or positions 34 to 50 of SEQ ID NO: 27;
      FR3 consisting of an amino acid sequence having 70% or more sequence identity with an amino acid sequence at positions 59 to 95 of SEQ ID NO: 21, positions 60 to 96 of SEQ ID NO: 23, positions 59 to 95 of SEQ ID NO: 25, or positions 60 to 96 of SEQ ID NO: 27; and
      FR4 consisting of an amino acid sequence having 70% or more sequence identity with an amino acid sequence at positions 109 to 119 of SEQ ID NO: 21, positions 109 to 119 of SEQ ID NO: 23, positions 107 to 117 of SEQ ID NO: 25, or positions 113 to 123 of SEQ ID NO: 27; and
   the light chain variable region includes:
      FR1 consisting of an amino acid sequence having 70% or more sequence identity with an amino acid sequence at positions 1 to 26 of SEQ ID NO: 22, positions 1 to 26 of SEQ ID NO: 24, positions 1 to 26 of SEQ ID NO: 26, or positions 1 to 26 of SEQ ID NO: 28;
      FR2 consisting of an amino acid sequence having 70% or more sequence identity with an amino acid sequence at positions 33 to 49 of SEQ ID NO: 22, positions 33 to 49 of SEQ ID NO: 24, positions 33 to 49 of SEQ ID NO: 26, or positions 33 to 49 of SEQ ID NO: 28;
      FR3 consisting of an amino acid sequence having 70% or more sequence identity with an amino acid sequence at positions 53 to 88 of SEQ ID NO: 22, positions 53 to 88 of SEQ ID NO: 24, positions 53 to 88 of SEQ ID NO: 26, or positions 53 to 88 of SEQ ID NO: 28, and
      FR4 consisting of an amino acid sequence having 70% or more sequence identity with an amino acid sequence at positions 101 to 110 of SEQ ID NO: 22, positions 101 to 110 of SEQ ID NO: 24, positions 101 to 109 of SEQ ID NO: 26, or positions 98 to 107 of SEQ ID NO: 28.
Aspect 7: The anti-AAV full capsid molecule according to Aspect 2, wherein the heavy chain variable region includes an amino acid sequence set forth in SEQ ID NO: 21, and the light chain variable region includes an amino acid sequence set forth in SEQ ID NO: 22.
Aspect 8: The anti-AAV full capsid molecule according to Aspect 3, wherein the heavy chain variable region includes an amino acid sequence set forth in SEQ ID NO: 23, and the light chain variable region includes an amino acid sequence set forth in SEQ ID NO: 24.
Aspect 9: The anti-AAV full capsid molecule according to Aspect 4, wherein the heavy chain variable region includes an amino acid sequence set forth in SEQ ID NO: 25, and the light chain variable region includes an amino acid sequence set forth in SEQ ID NO: 26.
Aspect 10: The anti-AAV full capsid molecule according to Aspect 5, wherein the heavy chain variable region includes an amino acid sequence set forth in SEQ ID NO: 27, and the light chain variable region includes an amino acid sequence set forth in SEQ ID NO: 28.
Aspect 11: The anti-AAV full capsid molecule according to any one of Aspects 1 to 10, which is an IgG antibody, a Fab antibody, a Fab' antibody, a F(ab')² antibody, an Fv antibody, an scFv antibody, a dsFv antibody, an scFv-Fc antibody, a dsFv-Fc antibody, a Bis-scFv antibody, a minibody, a diabody, a triabody, or a tetrabody.
Aspect 12: An affinity solid phase for binding AAV full capsids, the affinity solid phase including the anti-AAV full capsid molecule described in any one of Aspects 1 to 11 and a solid-phase material with the anti-AAV full capsid molecule immobilized thereon.
Aspect 13: A method for capturing AAV full capsids, the method including a step 1 of bringing a sample containing AAV empty capsids and AAV full capsids into contact with the affinity solid phase for binding AAV full capsids described in Aspect 12, thereby capturing the AAV full capsids.
Aspect 14: The method according to Aspect 13, further including a step 2 of quantifying the captured AAV full capsids by enzyme-linked immunosorbent assay.
Aspect 15: A nucleic acid encoding the anti-AAV full capsid molecule described in any one of Aspects 1 to 11.
Aspect 16: An expression cassette or a recombinant vector containing the nucleic acid described in Aspect 15.
Aspect 17: A transformant obtained by transforming a host using the expression cassette or recombinant vector described in Aspect 16.
Aspect 18: A method for producing an anti-AAV full capsid molecule, the method including culturing the transformant described in Aspect 17.

### Advantageous Effects of Invention

According to the present disclosure, an anti-AAV full capsid molecule having higher binding affinity for AAV full capsids than for AAV empty capsids is provided. Therefore, according to the present disclosure, quantification of full capsids using the anti-AAV full capsid molecule becomes possible.

### Brief Description of Drawings

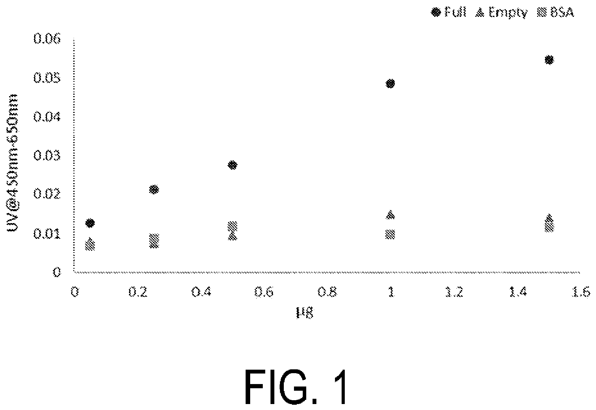

FIG. 1 is a diagram illustrating the relationship between the amount of AAV9 full capsids and the ELISA signal (absorbance) using the anti-AAV full capsid molecule (anti-AAV-scFv) of the present disclosure.

### Description of Embodiments

### 1. Anti-AAV Full Capsid Molecule

The anti-AAV full capsid molecule of the present disclosure is an antibody having higher binding affinity for AAV full capsids than for AAV empty capsids. That is, the anti-AAV full capsid molecule of the present disclosure is a polypeptide having higher binding affinity for AAV full capsids than for AAV empty capsids, and has a heavy chain variable region including heavy chain complementarity-determining regions that exhibit higher binding affinity for AAV full capsids than for AAV empty capsids, and a light chain variable region including light chain complementarity-determining regions. Preferably, the anti-AAV full capsid molecule of the present disclosure is a polypeptide having higher binding affinity for AAV full capsids than for AAV empty capsids, and has a heavy chain variable region including predetermined heavy chain complementarity-determining regions and a light chain variable region including predetermined light chain complementarity-determining regions.

### 1-1. Target

The AAV serotype to be targeted by the anti-AAV full capsid molecule of the present disclosure is not particularly limited. Examples of specific serotypes include AAV1, AAV2, AAV3a, AAV3b, AAV4, AAV5, AAV6, AAV7, AAV8, AAV9, AAV10, AAVrh.10, AAV11, AAV12, and AAV13, and AAV9 is preferred.

An AAV empty capsid is an empty outer shell of AAV that does not contain nucleic acids therein. A typical example of an AAV empty capsid is a vesicle used to package a target gene (foreign gene) that needs to be delivered to cells to be treated.

An AAV full capsid is an AAV capsid that contains nucleic acids therein. Examples of such nucleic acids include the viral genome of the AAV, nucleic acid drugs (such as antisense oligonucleotides, RNAi, aptamers, and decoys), and vectors with genes for gene therapy introduced therein.

### 1-2. Binding Affinity

The anti-AAV full capsid molecule of the present disclosure has higher binding affinity for AAV full capsids than for AAV empty capsids.

Higher binding affinity for AAV full capsids than for AAV empty capsids can be confirmed by the fact that the amount of binding of AAV full capsids is greater than the amount of binding of AAV empty capsids when the anti-AAV full capsid molecule of the present disclosure is brought into contact with AAV empty capsids or AAV full capsids under the same conditions. For example, the confirmation can be performed by preparing the anti-AAV full capsid molecule of the present disclosure in a form with an affinity tag, bringing the anti-AAV full capsid molecule into contact with AAV empty capsids or AAV full capsids under the same conditions, then capturing the anti-AAV full capsid molecule bound to the capsid with an antibody (anti-affinity tag antibody) specific to the affinity tag and having a signal group (such as a fluorescent group), and measuring the signal intensity based on the signal group of the anti-affinity tag antibody that has captured the anti-AAV full capsid molecule (such as the absorbance based on the fluorescent group).

As specific degree of binding affinity for AAV full capsids, a binding affinity for AAV full capsids based on the above absorbance when the anti-AAV full capsid molecule and AAV capsids are brought into contact at pH 7 (25°C) and 25°C for 1 hour is 1.5-fold or more, and specifically from 1.5 to 5-fold, preferably from 2 to 5-fold, more preferably from 2.5 to 5-fold, still more preferably from 3 to 5-fold or from 3.5 to 5-fold, and even more preferably from 4 to 5-fold, relative to the binding affinity for AAV empty capsids.

### 1-3. Complementarity-Determining Regions

The anti-AAV full capsid molecule of the present disclosure typically includes three heavy chain complementarity-determining regions (CDR1, CDR2, and CDR3 from the N-terminus side) and three light chain complementarity-determining regions (CDR1, CDR2, and CDR3 from the N-terminus side).

In a preferred embodiment, the anti-AAV full capsid molecule of the present disclosure includes a first anti-AAV full capsid molecule shown in [1] of Table 1, a second anti-AAV full capsid molecule shown in [2], a third anti-AAV full capsid molecule shown in [3], and a fourth anti-AAV full capsid molecule shown in [4].

**[Table 1]**

| | | CDR1 | CDR2 | CDR3 |
|---|---|---|---|---|
| [1] | Heavy chain | GFSFSSYG (SEQ IDNO: 1) | IYGGSSGST (SEQ ID NO: 2) | ARDRVGGNGYADL (SEQ ID NO: 3) |
| | Light chain | QSISSY (SEQ ID NO: 4) | RAS | QSHYDIRNYGVA (SEQ ID NO: 5) |
| [2] | Heavy chain | GFSFSSNYW (SEQ ID NO: 6) | IYGGSSGST (SEQ ID NO: 7) | ATVDNGYSDLKL (SEQ ID NO: 8) |
| | Light chain | QSIRNW (SEQ ID NO: 9) | RAS | QSSFYADSGEPA (SEQ ID NO: 10) |
| [3] | Heavy chain | GIDFSSGYD (SEQ IDNO: 11) | IYTGGDSA (SEQ ID NO: 12) | ANDLDSGTVSI (SEQ ID NO: 13) |
| | Light chain | QSISSW (SEQ ID NO: 14) | QAS | AGWTSSTTDGTA (SEQ ID NO: 15) |
| [4] | Heavy chain | GFSFSSSAY (SEQ ID NO: 16) | MHTISSVDT (SEQ ID NO: 17) | ARDDRNGYGYPYSFDI (SEQ ID NO: 18) |
| | Light chain | QTISSY (SEQ ID NO: 19) | RAS | LTYYGNSHN (SEQ ID NO: 20) |

The first anti-AAV full capsid molecule includes a heavy chain variable region including, as heavy chain complementarity-determining regions, CDR1 consisting of the amino acid sequence set forth in SEQ ID NO: 1, CDR2 consisting of the amino acid sequence set forth in SEQ ID NO: 2, and CDR3 consisting of an amino acid sequence having 90% or more sequence identity with the amino acid sequence set forth in SEQ ID NO: 3; and a light chain variable region including, as light chain complementarity-determining regions, CDR1 consisting of the amino acid sequence set forth in SEQ ID NO: 4, CDR2 consisting of RAS, and CDR3 consisting of an amino acid sequence having 90% or more sequence identity with the amino acid sequence set forth in SEQ ID NO: 5.

The second anti-AAV full capsid molecule includes a heavy chain variable region including, as heavy chain complementarity-determining regions, CDR1 consisting of the amino acid sequence set forth in SEQ ID NO: 6, CDR2 consisting of the amino acid sequence set forth in SEQ ID NO: 7, and CDR3 consisting of an amino acid sequence having 90% or more sequence identity with the amino acid sequence set forth in SEQ ID NO: 8; and a light chain variable region including, as light chain complementarity-determining regions, CDR1 consisting of the amino acid sequence set forth in SEQ ID NO: 9, CDR2 consisting of RAS, and CDR3 consisting of an amino acid sequence having 90% or more sequence identity with the amino acid sequence set forth in SEQ ID NO: 10. Among the anti-AAV full capsid molecules of the present disclosure, the second anti-AAV full capsid molecule is preferred in that it exhibits particularly high binding specificity for AAV full capsids.

The third anti-AAV full capsid molecule includes a heavy chain variable region including, as heavy chain complementarity-determining regions, CDR1 consisting of the amino acid sequence set forth in SEQ ID NO: 11, CDR2 consisting of the amino acid sequence set forth in SEQ ID NO: 12, and CDR3 consisting of an amino acid sequence having 90% or more sequence identity with the amino acid sequence set forth in SEQ ID NO: 13; and a light chain variable region including, as light chain complementarity-determining regions, CDR1 consisting of the amino acid sequence set forth in SEQ ID NO: 14, CDR2 consisting of QAS, and CDR3 consisting of an amino acid sequence having 90% or more sequence identity with the amino acid sequence set forth in SEQ ID NO: 15.

The fourth anti-AAV full capsid molecule includes a heavy chain variable region including, as heavy chain complementarity-determining regions, CDR1 consisting of the amino acid sequence set forth in SEQ ID NO: 16, CDR2 consisting of the amino acid sequence set forth in SEQ ID NO: 17, and CDR3 consisting of an amino acid sequence having 90% or more sequence identity with the amino acid sequence set forth in SEQ ID NO: 18; and a light chain variable region including, as light chain complementarity-determining regions, CDR1 consisting of the amino acid sequence set forth in SEQ ID NO: 19, CDR2 consisting of RAS, and CDR3 consisting of the amino acid sequence set forth in SEQ ID NO: 20.

In the present invention, the term "sequence identity" refers to a value of amino acid sequence identity obtained by the bl2seq program (Tatiana A. Tatsusova, Thomas L. Madden, FEMS Microbiol. Lett., Vol. 174, p247-250, 1999) of the BLAST PACKAGE [sgi32 bit edition, Version 2.0.12; available from National Center for Biotechnology Information (NCBI)]. The parameters may be set to a gap insertion cost value of 11 and a gap extension cost value of 1.

In an amino acid sequence having the above sequence identity of less than 100%, when mismatched amino acid residues are substituted with amino acids different from those in a reference amino acid sequence, such substitutions are preferably substitutions with similar amino acids (i.e., conservative amino acid substitutions). Specifically, the following classifications have been established based on the properties of amino acid side chains, and substitution with an amino acid belonging to the same classification is preferred.
Basic amino acids: lysine, arginine, histidine
Acidic amino acids: glutamic acid, aspartic acid
Neutral amino acids: glycine, alanine, serine, threonine, methionine, cysteine, phenylalanine, tryptophan, tyrosine, leucine, isoleucine, valine, glutamine, asparagine, proline

Furthermore, the neutral amino acids can also be classified, for example, into those having a polar side chain (asparagine, glutamine, serine, threonine, tyrosine, cysteine), those having a nonpolar side chain (glycine, alanine, valine, leucine, isoleucine, proline, phenylalanine, methionine, tryptophan), those having an amide-containing side chain (asparagine, glutamine), those having a sulfur-containing side chain (methionine, cysteine), those having an aromatic side chain (phenylalanine, tryptophan, tyrosine), those having a hydroxyl group-containing side chain (serine, threonine, tyrosine), those having an aliphatic side chain (alanine, leucine, isoleucine, valine).

As methods for substituting a predetermined amino acid in an amino acid sequence with another amino acid, for example, site-directed mutagenesis methods (such as Hashimoto-Gotoh T. et al., Gene, Vol. 152, p. 271-275 (1995); Zoller MJ. et al., Methods Enzymol. Vol. 100, p. 468-500 (1983); Kramer W. et al., Nucleic Acids Res. Vol. 12, p. 9441-9456 (1984); Kramer W. et al., Methods Enzymol. Vol. 154, p. 350-367 (1987); and Kunkel TA., Proc Natl Acad Sci USA., Vol. 82, p. 488-492 (1985)) are known, and amino acid substitutions can be performed on the amino acid sequence of CDRs using such site-directed mutagenesis methods. Examples of methods for substitution with another amino acid also include the library technology described in WO 2005/080432.

### 1-4. Framework Regions

The framework regions of the heavy chain variable regions and the light chain variable regions of the first to fourth anti-AAV full capsid molecules are not particularly limited as long as the anti-AAV full capsid molecules have higher binding affinity for AAV full capsids than for AAV empty capsids, and framework regions (FRs) of antibodies of any animal (human or non-human, preferably rabbit) can be used.

For example, the first to fourth anti-AAV full capsid molecules are preferably such that
the heavy chain variable region includes:
   FR1 consisting of an amino acid sequence having 70% or more sequence identity with the amino acid sequence at positions 1 to 24 of SEQ ID NO: 21, positions 1 to 24 of SEQ ID NO: 23, positions 1 to 24 of SEQ ID NO: 25, or positions 1 to 24 of SEQ ID NO: 27;
   FR2 consisting of an amino acid sequence having 70% or more sequence identity with the amino acid sequence at positions 33 to 49 of SEQ ID NO: 21, positions 34 to 50 of SEQ ID NO: 23, positions 34 to 50 of SEQ ID NO: 25, or positions 34 to 50 of SEQ ID NO: 27;
   FR3 consisting of an amino acid sequence having 70% or more sequence identity with the amino acid sequence at positions 59 to 95 of SEQ ID NO: 21, positions 60 to 96 of SEQ ID NO: 23, positions 59 to 95 of SEQ ID NO: 25, or positions 60 to 96 of SEQ ID NO: 27; and
   FR4 consisting of an amino acid sequence having 70% or more sequence identity with the amino acid sequence at positions 109 to 119 of SEQ ID NO: 21, positions 109 to 119 of SEQ ID NO: 23, positions 107 to 117 of SEQ ID NO: 25, or positions 113 to 123 of SEQ ID NO: 27; and
the light chain variable region includes:
   FR1 consisting of an amino acid sequence having 70% or more sequence identity with the amino acid sequence at positions 1 to 26 of SEQ ID NO: 22, positions 1 to 26 of SEQ ID NO: 24, positions 1 to 26 of SEQ ID NO: 26, or positions 1 to 26 of SEQ ID NO: 28;
   FR2 consisting of an amino acid sequence having 70% or more sequence identity with the amino acid sequence at positions 33 to 49 of SEQ ID NO: 22, positions 33 to 49 of SEQ ID NO: 24, positions 33 to 49 of SEQ ID NO: 26, or positions 33 to 49 of SEQ ID NO: 28;
   FR3 consisting of an amino acid sequence having 70% or more sequence identity with the amino acid sequence at positions 53 to 88 of SEQ ID NO: 22, positions 53 to 88 of SEQ ID NO: 24, positions 53 to 88 of SEQ ID NO: 26, or positions 53 to 88 of SEQ ID NO: 28, and
   FR4 consisting of an amino acid sequence having 70% or more sequence identity with the amino acid sequence at positions 101 to 110 of SEQ ID NO: 22, positions 101 to 110 of SEQ ID NO: 24, positions 101 to 109 of SEQ ID NO: 26, or positions 98 to 107 of SEQ ID NO: 28;
and are molecules having higher binding affinity for AAV full capsids than for AAV empty capsids.

Preferred examples of the above sequence identity of 70% or more may vary depending on the full length of the reference amino acid sequence, but include preferably 80% or more or 85% or more, more preferably 90% or more, still more preferably 92% or more, even more preferably 94% or more or 95% or more, yet more preferably 96% or more or 97% or more, and most preferably 100%.

### 1-5. Specific Examples of Variable Regions

More preferably, in the first anti-AAV full capsid molecule, the heavy chain variable region includes the amino acid sequence set forth in SEQ ID NO: 21, and the light chain variable region includes the amino acid sequence set forth in SEQ ID NO: 22.

SEQ ID NO: 21 of the heavy chain variable region is composed of, from the N terminus, {FR1 consisting of the amino acid sequence at positions 1 to 24} - {CDR1 consisting of the amino acid sequence at positions 25 to 32 (corresponding to CDR1 of SEQ ID NO: 1)} - {FR2 consisting of the amino acid sequence at positions 33 to 49} - {CDR2 consisting of the amino acid sequence at positions 50 to 58 (corresponding to CDR2 of SEQ ID NO: 2)} - {FR3 consisting of the amino acid sequence at positions 59 to 95} - {CDR3 consisting of the amino acid sequence at positions 96 to 108 (corresponding to CDR3 of SEQ ID NO: 3)} - {FR4 consisting of the amino acid sequence at positions 109 to 119}. SEQ ID NO: 22 of the light chain variable region is composed of, from the N terminus, {FR1 consisting of the amino acid sequence at positions 1 to 26} - {CDR1 consisting of the amino acid sequence at positions 27 to 32 (corresponding to CDR1 of SEQ ID NO: 4)} - {FR2 consisting of the amino acid sequence at positions 33 to 49} - {CDR2 consisting of the amino acid sequence at positions 50 to 52} - {FR3 consisting of the amino acid sequence at positions 53 to 88 of SEQ ID NO: 22} - {CDR3 consisting of the amino acid sequence at positions 89 to 100 (corresponding to CDR3 of SEQ ID NO: 5)} - {FR4 consisting of the amino acid sequence at positions 101 to 110 of SEQ ID NO: 22}.

More preferably, in the second anti-AAV full capsid molecule, the heavy chain variable region includes the amino acid sequence set forth in SEQ ID NO: 23, and the light chain variable region includes the amino acid sequence set forth in SEQ ID NO: 24.

SEQ ID NO: 23 of the heavy chain variable region is composed of, from the N terminus, {FR1 consisting of the amino acid sequence at positions 1 to 24} - {CDR1 consisting of the amino acid sequence at positions 25 to 33 (corresponding to CDR1 of SEQ ID NO: 6)} - {FR2 consisting of the amino acid sequence at positions 34 to 50} - {CDR2 consisting of the amino acid sequence at positions 51 to 59 (corresponding to CDR2 of SEQ ID NO: 7)} - {FR3 consisting of the amino acid sequence at positions 60 to 96} - {CDR3 consisting of the amino acid sequence at positions 97 to 108 (corresponding to CDR3 of SEQ ID NO: 8)} - {FR4 consisting of the amino acid sequence at positions 109 to 119}. SEQ ID NO: 24 of the light chain variable region is composed of, from the N terminus, {FR1 consisting of the amino acid sequence at positions 1 to 26} - {CDR1 consisting of the amino acid sequence at positions 27 to 32 (corresponding to CDR1 of SEQ ID NO: 9)} - {FR2 consisting of the amino acid sequence at positions 33 to 49} - {CDR2 consisting of the amino acid sequence at positions 50 to 52} - {FR3 consisting of the amino acid sequence at positions 53 to 88} - {CDR3 consisting of the amino acid sequence at positions 89 to 100 (corresponding to CDR3 of SEQ ID NO: 10)} - {FR4 consisting of the amino acid sequence at positions 101 to 110}.

More preferably, in the third anti-AAV full capsid molecule, the heavy chain variable region includes the amino acid sequence set forth in SEQ ID NO: 25, and the light chain variable region includes the amino acid sequence set forth in SEQ ID NO: 26.

SEQ ID NO: 25 of the heavy chain variable region is composed of, from the N terminus, {FR1 consisting of the amino acid sequence at positions 1 to 24} - {CDR1 consisting of the amino acid sequence at positions 25 to 33 (corresponding to CDR1 of SEQ ID NO: 11)} - {FR2 consisting of the amino acid sequence at positions 34 to 50} - {CDR2 consisting of the amino acid sequence at positions 51 to 58 (corresponding to CDR2 of SEQ ID NO: 12)} - {FR3 consisting of the amino acid sequence at positions 59 to 95} - {CDR3 consisting of the amino acid sequence at positions 96 to 106 (corresponding to CDR3 of SEQ ID NO: 13)} - {FR4 consisting of the amino acid sequence at positions 107 to 117}. SEQ ID NO: 26 of the light chain variable region is composed of, from the N terminus, {FR1 consisting of the amino acid sequence at positions 1 to 26} - {CDR1 consisting of the amino acid sequence at positions 27 to 32 (corresponding to CDR1 of SEQ ID NO: 14)} - {FR2 consisting of the amino acid sequence at positions 33 to 49} - {CDR2 consisting of the amino acid sequence at positions 50 to 52} - {FR3 consisting of the amino acid sequence at positions 53 to 88} - {CDR3 consisting of the amino acid sequence at positions 89 to 100 (corresponding to CDR3 of SEQ ID NO: 15)} - {FR4 consisting of the amino acid sequence at positions 101 to 109}.

More preferably, in the fourth anti-AAV full capsid molecule, the heavy chain variable region includes the amino acid sequence set forth in SEQ ID NO: 27, and the light chain variable region includes the amino acid sequence set forth in SEQ ID NO: 28.

SEQ ID NO: 27 of the heavy chain variable region is composed of, from the N terminus, {FR1 consisting of the amino acid sequence at positions 1 to 24} - {CDR1 consisting of the amino acid sequence at positions 25 to 33 (corresponding to CDR1 of SEQ ID NO: 16)} - {FR2 consisting of the amino acid sequence at positions 34 to 50 of SEQ ID NO: 27} - {CDR2 consisting of the amino acid sequence at positions 51 to 59 (corresponding to CDR2 of SEQ ID NO: 17)} - {FR3 consisting of the amino acid sequence at positions 60 to 96 of SEQ ID NO: 27} - {CDR3 consisting of the amino acid sequence at positions 97 to 112 (corresponding to CDR3 of SEQ ID NO: 18)} - {FR4 consisting of the amino acid sequence at positions 113 to 123 of SEQ ID NO: 27}. SEQ ID NO: 28 of the light chain variable region is composed of, from the N terminus, {FR1 consisting of the amino acid sequence at positions 1 to 26} - {CDR1 consisting of the amino acid sequence at positions 27 to 32 (corresponding to CDR1 of SEQ ID NO: 19)} - {FR2 consisting of the amino acid sequence at positions 33 to 49} - {CDR2 consisting of the amino acid sequence at positions 50 to 52} - {FR3 consisting of the amino acid sequence at positions 53 to 88} - {CDR3 consisting of the amino acid sequence at positions 89 to 97 (corresponding to CDR3 of SEQ ID NO: 20)} - {FR4 consisting of the amino acid sequence at positions 98 to 107}.

### 1-6. Specific Embodiments of Anti-AAV Full Capsid Molecule

The anti-AAV full capsid molecule of the present disclosure may be a complete antibody or a low-molecular-weight antibody (an antibody having a smaller molecular weight than a complete antibody).

When the anti-AAV full capsid molecule of the present disclosure is a complete antibody, the isotype thereof is not particularly limited, and examples thereof include IgG (IgG1, IgG2, IgG3, IgG4), IgA (IgA1, IgA2), IgM, IgD, and IgE.

When the anti-AAV full capsid molecule of the present disclosure is a low-molecular-weight antibody, specific examples thereof include a Fab antibody (about 55 kDa), a Fab' antibody (about 55 kDa), an F(ab')² antibody (about 110 kDa), an Fv antibody (about 25 kDa), an scFv antibody (about 25 kDa), a dsFv antibody (about 25 kDa), an scFv-Fc antibody (about 105 kDa), a dsFv-Fc antibody (about 105 kDa), a Bis-scFv antibody (about 50 kDa), a minibody (about 80 kDa), a diabody (about 55 kDa), a triabody (about 75 kDa), and a tetrabody (about 100kDa).

Furthermore, the anti-AAV full capsid molecule of the present disclosure also encompasses multispecific antibodies (e.g., bispecific antibodies) as long as it has a heavy chain variable region including predetermined heavy chain complementarity-determining regions and a light chain variable region including predetermined light chain complementarity-determining regions. These antibody fragments and multispecific antibodies can be produced according to known methods.

The anti-AAV full capsid molecule of the present disclosure is typically an isolated monoclonal antibody. The method for producing the monoclonal antibody is not particularly limited, and the monoclonal antibody can be produced by, for example, a hybridoma method as described in "Kohler G., Milstein C., Nature, Aug. 7, 1975; 256(5517): 495-497"; a recombinant method as described in U.S. Patent No. 4816567; a method of isolating from a phage antibody library as described in "Clackson et al., Nature, Aug. 15, 1991; 352(6336): 624-628" or "Marks et al., J Mol Biol., Dec. 5, 1991; 222(3): 581-597", or methods described in "Protein Experimental Handbook, Yodosha (2003): 92-96".

### 1-7. Other Components

The anti-AAV full capsid molecule of the present disclosure may include another component in addition to antibody constituent elements (the above complementarity-determining regions, the above frameworks, and portions other than the complementarity-determining regions and the frameworks constituting the above "1-6. Specific Embodiments of Anti-AAV Full Capsid Molecule"), as long as the anti-AAV full capsid molecule has higher binding affinity for AAV full capsids than for AAV empty capsids.

Examples of the anti-AAV full capsid molecule of the present disclosure that includes the other component include conjugate antibodies in which various compounds such as polyethylene glycol, radioactive substances, and toxins are bound as the other components; antibodies in which modified sugar chains are bound as the other component; and fusion antibodies in which another protein is fused as the other component.

Preferred examples of other components include hinge regions, spacers, purification tags for enabling purification of the anti-AAV full capsid molecule of the present disclosure, and immobilization modifying groups for enabling immobilization of the anti-AAV full capsid molecule of the present disclosure onto a solid phase. These components may be used alone or in combination of two or more.

The number of amino acid residues constituting the hinge region is not particularly limited, and examples thereof include from 1 to 25 aa, and preferably from 5 to 20 aa. Specific examples of the hinge region include EPKTPKPQ, AHHSEDPS, and EPTPPQPQPQPQPQPNPTTE.

As the spacer, a sequence having an appropriate length may be selected, and examples thereof include from 1 to 6 amino acids, and preferably from 2 to 5 amino acids.

The purification tag is bound to an antibody constituent element directly or indirectly via an optional linker group. Examples of the purification tags include epitope tags such as a histidine tag (an oligo-histidine consisting of 6 or more histidine residues, preferably from 6 to 10 residues, which reversibly chelates to a solid phase having, on its surface, metal ions serving as central metals such as nickel (Ni²⁺) or cobalt (Co²⁺)); a Strep-tag (a tag consisting of WSHPQFEK, which reversibly binds to a solid phase having streptavidin on its surface); a FLAG tag (a tag consisting of DYKDDDDK or DYKDHD-G-DYKDHD-I-DYKDDDDK, which reversibly binds to a solid phase having a FLAG-tag recognition site on its surface); a Spot-tag (a tag consisting of PDRVRAVSHWSS, which reversibly binds to a solid phase having a Spot-tag recognition site on its surface); a C-tag (a tag consisting of EPEA, which reversibly binds to a solid phase having a C-tag recognition site on its surface); and a Myc tag (a tag consisting of EQKLISEEDL, which reversibly binds to a solid phase having an anti-Myc tag antibody on its surface); and protein tags such as a glutathione S-transferase tag (which reversibly binds to a solid phase having a glutathione S-transferase recognition site or glutathione on its surface) and a maltose-binding protein tag (which reversibly binds to a solid phase having a maltose-binding protein recognition site on its surface). As the other components, these purification tags may be used alone or in combination of two or more. When two or more of these purification tags are used in combination, the purification tags can be linked via an appropriate spacer.

The immobilization modifying group is bound to an antibody constituent element directly or indirectly via an optional linker group. Examples of the immobilization modifying groups include an amino group (a group that forms a carbamoyl group together with a carboxyl group of a polypeptide, which is immobilized via an amino group onto a solid phase having an active ester group or an epoxy group immobilized on its surface); a thiol group (immobilized onto a solid phase whose surface is modified with a maleimide group by a Michael addition reaction); a cyclopentadienyl group (immobilized by a Diels-Alder reaction onto a solid phase whose surface is modified with a quinone group); a biotinyl group (immobilized via a biotin-avidin interaction onto a solid phase whose surface is modified with an avidin group); an oxyamino group (immobilized via a Schiff base onto a solid phase whose surface is modified with a formyl group); a cysteine residue (immobilized via thiazoline ring formation onto a solid phase whose surface is modified with a formyl group, or immobilized by transesterification onto a solid phase whose surface is modified with a benzyl thioester); and a group that modifies a carboxyl group using 2-(2-pyridinyldithio)ethanamine (PDEA) (immobilized via a disulfide bond onto a solid phase whose surface is modified with a thiol group).

The above purification tags or immobilization modifying groups can be introduced into antibody constituent elements by known methods, thereby enabling synthesis of the anti-AAV full capsid molecule of the present disclosure.

### 2. Affinity Solid Phase for Binding AAV Full Capsids

The affinity solid phase for binding AAV full capsids of the present disclosure includes the above "1. Anti-AAV Full Capsid Molecule" and a solid-phase material (also simply referred to as "solid phase" in the present specification) with the anti-AAV full capsid molecule immobilized thereon.

The material of the solid-phase material is not particularly limited, and examples thereof include resins (such as agarose, sepharose, dextran, silica gel, polyacrylamide, polystyrene, polyethylene, polypropylene, polyester, polyacrylonitrile, (meth)acrylic acid-based polymers, fluororesins, and metal complex resins), glass, metals, and magnetic materials.

The form of the solid-phase material is not particularly limited, and can be appropriately determined according to the mode of use of the affinity solid phase for binding AAV full capsids of the present disclosure. Examples of the form of the solid phase include a flat plate shape, a particulate form, and a fibrous form.

When the form of the solid-phase material is a flat plate shape, the flat plate-shaped solid phase may constitute a chip, or may constitute the bottom surface of, for example, a container or a well. When the form of the solid-phase material is particulate or fibrous, the particulate or fibrous solid phase may be in a form packed in a column.

In the affinity solid phase for binding AAV full capsids of the present disclosure, the mode of immobilization of the anti-AAV full capsid molecule is as described in the description of the immobilization modifying groups of the above "1-7. Other Components".

### 3. Method for Capturing AAV Full Capsids

The method for capturing AAV full capsids of the present disclosure includes a step 1 of bringing a sample containing AAV empty capsids and AAV full capsids into contact with the affinity solid phase for binding AAV full capsids described above in "2. Affinity Solid Phase for Binding AAV Full Capsids", thereby capturing the AAV full capsids.

Since the anti-AAV full capsid molecule immobilized on the affinity solid phase for binding AAV full capsids exhibits higher binding affinity for AAV full capsids than for AAV empty capsids, in step 1, the sample containing AAV empty capsids and AAV full capsids is brought into contact with the affinity solid phase, thereby selectively capturing AAV full capsids in the sample. The conditions for contact of the sample with the solid phase are not particularly limited, and examples thereof include neutral conditions (a pH at 25°C, for example, from 6.5 to 7.7), and for example, at normal temperature (preferably from 15 to 30°C), for example, for 1 minute to 3 hours.

The method for capturing AAV full capsids of the present disclosure can be used, for example, in a method for quantifying AAV full capsids in the above sample or a method for separating AAV full capsids in the above sample.

When the method for capturing AAV full capsids of the present disclosure is used in a method for quantifying AAV full capsids in the above sample, the method can further include a step 2 of quantifying the captured AAV full capsids by enzyme-linked immunosorbent assay. As a method for the enzyme-linked immunosorbent assay, a known method can be used, specifically, any of a direct method, an indirect method, a sandwich method, or a competitive method. In step 2, the binding between the affinity solid phase and the AAV full capsids can be detected based on a signal emitted by a signal group such as a fluorescent label, and therefore, the AAV full capsids can be quantified based on the intensity of the signal.

When the method for capturing AAV full capsids of the present disclosure is used in a method for separating AAV full capsids in the above sample, the method can further include a step 3 of separating and collecting the captured AAV full capsids. In this case, the solid-phase material can be used in a form packed in a chromatography column. In step 3, the AAV full capsids captured on the affinity solid phase are collected by elution. Examples of elution methods include elution by adjustment of pH (for example, acid elution or alkali elution) and elution by adjustment of salt concentration (for example, elution with a high salt concentration). The collected AAV full capsid fraction can be further purified by any purification method as necessary. The collected AAV full capsid fraction or a purified fraction thereof may be produced into a powder by, for example, freeze-drying, vacuum drying, spray drying as necessary.

### 4. Nucleic Acid

A nucleic acid encoding the anti-AAV full capsid molecule described in the above "1. Anti-AAV Full Capsid Molecule" (hereinafter, also referred to as "the nucleic acid of the present disclosure") can be appropriately prepared and designed by those skilled in the art according to the amino acid sequence of the anti-AAV full capsid molecule of the present disclosure. The nucleic acid of the present disclosure may be DNA or RNA.

The nucleotide sequence of the nucleic acid of the present disclosure can be appropriately designed by those skilled in the art according to the amino acid sequences of the complementarity-determining regions described in the above "1-3. Complementarity-Determining Regions".

For example, in a nucleotide sequence encoding the first anti-AAV full capsid molecule, examples of nucleotide sequences encoding CDR1 set forth in SEQ ID NO: 1, CDR2 set forth in SEQ ID NO: 2, and CDR3 set forth in SEQ ID NO: 3 include DNA consisting of the nucleotide sequences set forth in SEQ ID NOs: 29, 30, and 31, and nucleotide sequences complementary thereto, and DNA nucleotide sequences that hybridize under stringent conditions thereto. Examples of nucleotide sequences encoding CDR1 set forth in SEQ ID NO: 4, CDR2 consisting of RAS, and CDR3 set forth in SEQ ID NO: 5 include DNA consisting of the nucleotide sequence set forth in SEQ ID NO: 32, agggcatcc, and the nucleotide sequence set forth in SEQ ID NO: 33, and nucleotide sequences complementary thereto, and DNA nucleotide sequences that hybridize under stringent conditions thereto.

In a nucleotide sequence encoding the second anti-AAV full capsid molecule, examples of nucleotide sequences encoding CDR1 set forth in SEQ ID NO: 6, CDR2 set forth in SEQ ID NO: 7, and CDR3 set forth in SEQ ID NO: 8 include DNA consisting of the nucleotide sequences set forth in SEQ ID NOs: 34, 35, and 36, and nucleotide sequences complementary thereto, and DNA nucleotide sequences that hybridize under stringent conditions thereto. Examples of nucleotide sequences encoding CDR1 set forth in SEQ ID NO: 9, CDR2 consisting of RAS, and CDR3 set forth in SEQ ID NO: 10 include DNA consisting of the nucleotide sequence set forth in SEQ ID NO: 37, agggcatcc, and the nucleotide sequence set forth in SEQ ID NO: 38, and nucleotide sequences complementary thereto, and DNA nucleotide sequences that hybridize under stringent conditions thereto.

In a nucleotide sequence encoding the third anti-AAV full capsid molecule, examples of nucleotide sequences encoding CDR1 set forth in SEQ ID NO: 11, CDR2 set forth in SEQ ID NO: 12, and CDR3 set forth in SEQ ID NO: 13 include DNA consisting of the nucleotide sequences set forth in SEQ ID NOs: 39, 40, and 41, and nucleotide sequences complementary thereto, and DNA nucleotide sequences that hybridize under stringent conditions thereto. Examples of nucleotide sequences encoding CDR1 set forth in SEQ ID NO: 14, CDR2 consisting of QAS, and CDR3 set forth in SEQ ID NO: 15 include DNA consisting of the nucleotide sequence set forth in SEQ ID NO: 42, caggcatcc, and the nucleotide sequence set forth in SEQ ID NO: 43, and nucleotide sequences complementary thereto, and DNA nucleotide sequences that hybridize under stringent conditions thereto.

In a nucleotide sequence encoding the fourth anti-AAV full capsid molecule, examples of nucleotide sequences encoding CDR1 set forth in SEQ ID NO: 16, CDR2 set forth in SEQ ID NO: 17, and CDR3 set forth in SEQ ID NO: 18 include DNA consisting of the nucleotide sequences set forth in SEQ ID NOs: 44, 45, and 46, and nucleotide sequences complementary thereto, and DNA nucleotide sequences that hybridize under stringent conditions thereto. Examples of nucleotide sequences encoding CDR1 set forth in SEQ ID NO: 19, CDR2 consisting of RAS, and CDR3 set forth in SEQ ID NO: 20 include DNA consisting of the nucleotide sequence set forth in SEQ ID NO: 47, agggcatcc, and the nucleotide sequence set forth in SEQ ID NO: 48, and nucleotide sequences complementary thereto, and DNA nucleotide sequences that hybridize under stringent conditions thereto.

The nucleotide sequence of the nucleic acid of the present disclosure can further be appropriately designed by those skilled in the art according to the amino acid sequences of the framework regions described in the above "1-4. Framework Regions" or the amino acid sequences of the variable regions described in the above "1-5. Specific Examples of Variable Regions". For example, in a nucleotide sequence encoding the first anti-AAV full capsid molecule, examples of nucleotide sequences encoding the heavy chain variable region and the light chain variable region include DNA consisting of the nucleotide sequences set forth in SEQ ID NOs: 49 and 50, which encode the amino acid sequences set forth in SEQ ID NOs: 21 and 22, respectively, and nucleotide sequences complementary thereto, and DNA nucleotide sequences that hybridize under stringent conditions thereto. In a nucleotide sequence encoding the second anti-AAV full capsid molecule, examples of nucleotide sequences encoding the heavy chain variable region and the light chain variable region include DNA consisting of the nucleotide sequences set forth in SEQ ID NOs: 51 and 52, which encode the amino acid sequences set forth in SEQ ID NOs: 23 and 24, respectively, and nucleotide sequences complementary thereto, and DNA nucleotide sequences that hybridize under stringent conditions thereto. In a nucleotide sequence encoding the third anti-AAV full capsid molecule, examples of nucleotide sequences encoding the heavy chain variable region and the light chain variable region include DNA consisting of the nucleotide sequences set forth in SEQ ID NOs: 53 and 54, which encode the amino acid sequences set forth in SEQ ID NOs: 25 and 26, respectively, and nucleotide sequences complementary thereto, and DNA nucleotide sequences that hybridize under stringent conditions thereto. In a nucleotide sequence encoding the fourth anti-AAV full capsid molecule, examples of nucleotide sequences encoding the heavy chain variable region and the light chain variable region include DNA consisting of the nucleotide sequences set forth in SEQ ID NOs: 55 and 56, which encode the amino acid sequences set forth in SEQ ID NOs: 27 and 28, respectively, and nucleotide sequences complementary thereto, and DNA nucleotide sequences that hybridize under stringent conditions thereto.

The term "stringent conditions" refers to conditions in which incubation is carried out at 50°C to 65°C for 4 hours to overnight in 6× SSC (1× SSC is 0.15 M NaCl and 0.015 M sodium citrate, pH 7.0) containing 0.5% SDS, 5× Denhardt's solution [0.1% bovine serum albumin (BSA), 0.1% polyvinylpyrrolidone, and 0.1% Ficoll 400], and 100 µg/mL salmon sperm DNA. Specifically, hybridization under stringent conditions is carried out by the following method. That is, a nylon membrane on which a DNA library or a cDNA library is immobilized is prepared, and the nylon membrane is blocked at 65°C in a prehybridization solution containing 6× SSC, 0.5% SDS, 5× Denhardt's solution, and 100 µg/mL salmon sperm DNA. Then, each probe labeled with ³²P is added and incubated overnight at 65°C. The nylon membrane is washed in 6× SSC at room temperature for 10 minutes, in 2× SSC containing 0.1% SDS at room temperature for 10 minutes, and in 0.2× SSC containing 0.1% SDS at 45°C for 30 minutes, and then autoradiographed to detect DNA specifically hybridized with the probe.

The nucleotide sequence of the nucleic acid of the present disclosure can further be appropriately designed in accordance with those of the other components described in the above "1-7. Other Components" that are represented by amino acid sequences.

The nucleic acid of the present disclosure can also be obtained by obtaining at least a region encoding the above anti-AAV full capsid molecule by, for example, PCR, using a nucleic acid encoding the anti-AAV full capsid molecule as a template. The nucleic acid of the present disclosure can also be artificially synthesized by a gene synthesis method.

The nucleic acid of the present disclosure may further include at least one nucleotide sequence encoding an initiation codon or a stop codon.

Examples of methods for obtaining the nucleic acid of the present disclosure include hybridization-based methods.

The nucleic acid of the present disclosure encompasses various nucleic acids derived from codon degeneracy. Artificial production of various nucleic acids encoding the same amino acid sequence can be easily performed using known genetic engineering techniques. For example, in the genetic engineering-based production of polypeptides, when codons used in the native gene encoding a target protein are codons that are infrequently used in a host, the expression level of the protein may be low. In such cases, the target protein can be expressed at a high level by optimizing the codon usage frequency for the host without changing the encoded amino acid sequence.

As an index representing the codon usage frequency, the total of the host-optimal codon usage frequencies of the respective codons may be adopted. An optimal codon is defined as a codon having the highest usage frequency among codons corresponding to the same amino acid. The codon usage frequency is not particularly limited as long as it is optimized for the host, and examples of optimal codons for Escherichia coli include the following. F: phenylalanine (ttt), L: leucine (ctg), I: isoleucine (att), M: methionine (atg), V: valine (gtg), Y: tyrosine (tat), stop codon (taa), H: histidine (cat), Q: glutamine (cag), N: asparagine (aat), K: lysine (aaa), D: aspartic acid (gat), E: glutamic acid (gaa), S: serine (agc), P: proline (ccg), T: threonine (acc), A: alanine (gcg), C: cysteine (tgc), W: tryptophan (tgg), R: arginine (cgc), G: glycine (ggc).

### 5. Expression Cassette or Recombinant Vector

An expression cassette or recombinant vector containing the nucleic acid described in the above "4. Nucleic Acid" (hereinafter, also referred to as "the expression cassette of the present disclosure" or "the recombinant vector of the present disclosure") contains a nucleic acid encoding the polypeptide of the present disclosure.

The expression cassette or recombinant vector of the present disclosure can be obtained by ligating a promoter and a terminator to the nucleic acid of the present disclosure, or by inserting the expression cassette of the present disclosure or the nucleic acid of the present disclosure into an expression vector.

The expression cassette or recombinant vector of the present disclosure may further include, as regulatory factors, transcriptional elements such as an enhancer, a CCAAT box, a TATA box, and an SPI site, in addition to a promoter and a terminator, as necessary. These regulatory factors need only be operably linked to the DNA of the present disclosure. The term "operably linked" means that various regulatory factors that regulate the DNA of the present disclosure and the DNA of the present disclosure are linked in a state capable of functioning in a host cell.

When the expression cassette of the present disclosure or the recombinant vector of the present disclosure is designed so that a full-length polypeptide including a protease recognition sequence is first expressed and a terminal sequence can thereafter be cleaved by a protease, the expression cassette or recombinant vector of the present disclosure can be configured to include, in combination, a nucleotide sequence encoding the protease recognition sequence and a nucleotide sequence encoding an N-terminal sequence and/or a C-terminal sequence.

As the expression vector, vectors constructed for genetic recombination from a phage, a plasmid, or a virus capable of autonomously proliferating in a host are preferred. Such expression vectors are known, and a person skilled in the art can appropriately select and use an appropriate combination with a host cell. For example, when a microorganism is used as a host, examples include pBluescript (pBS) II SK(-) (available from Stratagene), pSTV-based vectors (available from Takara Bio), pUC-based vectors (available from Takara Bio), pET-based vectors (available from Sigma-Aldrich Japan), pGEX-based vectors (available from Global Life Science Technologies Japan (Cytiva)), pCold-based vectors (available from Takara Bio), pHY300PLK (available from Takara Bio), pUB110 (Mckenzie, T. et al., 1986, Plasmid 15(2), p. 93-103), pBR322 (available from Takara Bio), pRS403 (available from Stratagene), and pMW218/219 (available from Nippon Gene). When algae or microalgae are used as a host, examples include pUC19 (available from Takara Bio), P66 (Chlamydomonas Center), P-322 (Chlamydomonas Center), pPha-T1 (see Yangmin Gong, et al., Journal of Basic Microbiology, 2011, vol. 51, p. 666-672), and pJET1 (available from Thermo Fisher Scientific). When plant cells are used as a host, examples include pRI-based vectors (available from Takara Bio), pBI-based vectors (available from Clontech), and IN3-based vectors (available from Inplanta Innovations).

### 6. Transformant

A transformant (hereinafter, also referred to as "the transformant of the present disclosure") is obtained by transforming a host using the expression cassette or recombinant vector of the present disclosure.

The host used for producing the transformant is not particularly limited as long as it is capable of gene introduction, autonomous proliferation, and expression of the genetic traits of the present disclosure, and preferred examples thereof include microorganisms such as bacteria belonging to the genus Escherichia such as Escherichia coli, the genus Bacillus such as Bacillus subtilis, and the genus Pseudomonas such as Pseudomonas putida; actinomycetes; yeasts; and filamentous fungi, but in addition, animal cells, insect cells, plant cells may also be used. Among these, Escherichia coli is particularly preferred.

The transformant of the present disclosure can be obtained by introducing the expression cassette or recombinant vector of the present disclosure into a host. The site into which the nucleic acid of the present disclosure is introduced is also not particularly limited as long as the target gene can be expressed, and may be on a plasmid or on a genome. Specific examples of the method for introducing the expression cassette of the present disclosure or the recombinant vector of the present disclosure include a recombinant vector method and a genome editing method.

The conditions for introducing the expression cassette or recombinant vector of the present disclosure into a host may be appropriately set according to, for example, the type of host. When the host is a microorganism, examples of the method include a method using competent cells obtained by calcium ion treatment, an electroporation method, a spheroplast method, and a lithium acetate method. When the host is an animal cell, examples of the method include an electroporation method, a calcium phosphate method, and a lipofection method. When the host is an insect cell, examples of the method include a calcium phosphate method, a lipofection method, and an electroporation method. When the host is a plant cell, examples of the method include an electroporation method, an Agrobacterium method, a particle gun method, and a PEG method.

### 7. Method for Producing Anti-AAV Full Capsid Molecule

The present disclosure also provides a method for producing the anti-AAV full capsid molecule described in the above "1. Anti-AAV Full Capsid Molecule". The anti-AAV full capsid molecule, which is an antibody with higher binding affinity for AAV full capsids than for AAV empty capsids, can be appropriately produced by finite mechanical operations.

7-1.
   The anti-AAV full capsid molecule of the present disclosure can be appropriately obtained by finite mechanical operations including selecting antibodies from an antibody library by affinity selection for AAV full capsids and confirming that the selected antibodies have higher binding affinity for AAV full capsids than for AAV empty capsids. Specifically, an anti-AAV full capsid molecule can be obtained by immunizing an animal with an AAV capsid protein as an antigen, obtaining antibody genes from lymphocytes of the animal, and introducing the obtained antibody genes in which various VH regions and various VL regions are combined into a phagemid vector to construct an antibody library; performing biopanning to select phage clones having binding affinity for AAV full capsids; and confirming that the antibodies contained in the culture supernatant of the selected phage clones have higher binding affinity for AAV full capsids than for AAV empty capsids.
7-2.
   The anti-AAV full capsid molecule of the present disclosure can be appropriately produced by finite mechanical operations not only with CDRs consisting of the amino acid sequences set forth in specific SEQ ID NOs but also with CDRs having a sequence identity of 90% or more and less than 100% to the amino acid sequences set forth in the specific SEQ ID NOs. Specifically, an anti-AAV full capsid molecule of the present disclosure can be obtained by producing an antibody phage library by introducing, into a phagemid vector, antibody genes having, in the CDR regions on the structural domains, sequences of extremely limited variations (preferably variation sequences resulting from conservative amino acid substitutions) having a sequence identity of 90% or more and less than 100% to the amino acid sequences set forth in specific SEQ ID NOs; selecting phage clones having binding affinity for AAV full capsids by biopanning as described in the above item 7-1; and confirming that the antibodies produced by the selected phage clones have higher binding affinity for AAV full capsids than for AAV empty capsids.
7-3.
   In a preferred embodiment, the anti-AAV full capsid molecule of the present disclosure can be produced by culturing the transformant of the present disclosure.

The culture conditions for the transformant of the present disclosure may be appropriately set in consideration of the nutritional and physiological properties of the host, but liquid culture is preferred. In addition, in the case of industrial production, aerated and agitated culture is preferred.

The transformant of the present disclosure is cultured, and the culture supernatant, the cultured microbial cells, or the cultured cells are collected by a method such as centrifugation of the culture liquid. When the polypeptide of the present disclosure accumulates within cultured microbial cells or cultured cells, the microbial cells or cells are treated by mechanical methods such as sonication or a French press, or with a lytic enzyme such as lysozyme, and, as necessary, solubilized by using an enzyme such as a protease or a surfactant such as sodium dodecyl sulfate (SDS), thereby obtaining a water-soluble fraction containing the anti-AAV full capsid molecule of the present disclosure.

In addition, by selecting an appropriate expression vector and host, the expressed anti-AAV full capsid molecule of the present disclosure may be secreted into the culture liquid.

The culture liquid, water-soluble fraction, or protease-treated product containing the anti-AAV full capsid molecule of the present disclosure obtained as described above may be directly subjected to a purification treatment, or the anti-AAV full capsid molecule of the present disclosure in the culture liquid, water-soluble fraction, or protease-treated product may be concentrated and then subjected to a purification treatment.

The concentration can be carried out by, for example, concentration under reduced pressure, membrane concentration, salting-out treatment, or fractional precipitation using a hydrophilic organic solvent (e.g., methanol, ethanol and/or acetone).

The purification treatment of the anti-AAV full capsid molecule of the present disclosure can be performed, for example, by appropriately combining methods such as gel filtration, hydrophobic chromatography, ion-exchange chromatography, and affinity chromatography corresponding to a purification tag.

The anti-AAV full capsid molecule of the present disclosure purified in this manner may be converted into a powder by, for example, freeze-drying, vacuum drying, spray drying, as necessary.

Each aspect disclosed in the present specification can be combined with any other feature disclosed in the present specification.

### Examples

Hereinafter, the present invention will be more specifically described by examples, but each configuration, a combination of the configurations in each embodiment are merely examples, and additions, omissions, substitutions, and other changes of the configurations may appropriately be made without departing from the spirit of the present invention. The present disclosure is not limited by the embodiments and is limited only by the claims.

### [Test Example 1]

### (1) Preparation of Antibody Library

Rabbits were immunized using a protein excised from an AAV capsid protein as an antigen to induce antibody production. Total RNA was obtained from the spleen, and a cDNA library was constructed by reverse transcription polymerase chain reaction. Next, using primers, genes of the heavy chain (H-chain) variable region (VH domain) and genes of the light chain (L-chain) variable region (VL domain) were amplified by PCR. Next, in order to insert these PCR products into a phagemid vector, treatment with a restriction enzyme, for example, was performed. In addition, the phagemid vector was also treated with a predetermined restriction enzyme, and each gene treated with the restriction enzyme was inserted into the phagemid vector treated with the restriction enzyme. Escherichia coli TG-1 was transformed with this recombinant phagemid vector and inoculated into 10 mL of 2×YT medium (containing 1% glucose and 50 mg/L ampicillin). The cells were cultured overnight at 37°C and 200 rpm in a 200 mL Erlenmeyer flask (preculture).

The preculture liquid was inoculated in 50 mL of 2×YT medium (containing 1% glucose and 50 mg/mL ampicillin) to give an OD600 of 0.1, and shake-cultured at 30°C and 200 rpm. After culturing until an OD of approximately 1.0, helper phage VCSM13 was added to the culture liquid to give a multiplicity of infection (MOI) of 20, and the culture liquid was incubated at 37°C for 30 minutes. The culture liquid was centrifuged at 3000 × g and 37°C for 10 minutes, the supernatant was removed, and the cells were gently suspended in 50 mL of 2×YT medium (containing 50 mg/L ampicillin and 35 mg/L kanamycin). The culture was shaken at 30°C and 200 rpm for 12 hours or more to produce phages displaying single-chain antibodies in the culture supernatant. The supernatant was collected by centrifugation and concentrated by PEG precipitation, thereby obtaining a library of phages displaying single-chain antibodies in 1 mL of PBS.

### (2) Biopanning

An AAV9 full capsid sample was physically adsorbed to immunotubes in a buffer (137 mmol/L NaCl, 8.1 mmol/L Na₂HPO₄, 2.68 mmol/L KCl, 1.47 mmol/L KH₂PO₄, pH 7.4). Subsequently, each tube was blocked with a solution with BSA dissolved or dispersed in a concentration of 2% in a buffer (137 mmol/L NaCl, 8.1 mmol/L Na₂HPO₄, 2.68 mmol/L KCl, 1.47 mmol/L KH₂PO₄, pH 7.4). After incubation for a while, the tubes were washed three times with a buffer containing 0.1% Tween 20 (137 mmol/L NaCl, 8.1 mmol/L Na₂HPO₄, 2.68 mmol/L KCl, 1.47 mmol/L KH₂PO₄, pH 7.4).

The phage library prepared in the above (1) (about 1.0 × 10¹¹ pfu) was dissolved and dispersed in a buffer containing 2% BSA (137 mmol/L NaCl, 8.1 mmol/L Na₂HPO₄, 2.68 mmol/L KCl, 1.47 mmol/L KH₂PO₄, pH 7.4), added to the tubes, and incubated at 25°C for 1 hour. After incubation, the solution was removed, the tubes were washed five times with a buffer containing 0.1% Tween 20 (137 mmol/L NaCl, 8.1 mmol/L Na₂HPO₄, 2.68 mmol/L KCl, 1.47 mmol/L KH₂PO₄, pH 7.4), and 0.1 M glycine hydrochloride (glycine-HCl) (pH 2.2) was added, followed by incubation at 25°C for 10 minutes. Thereafter, the solution in the tubes was collected, neutralized, and then used to infect Escherichia coli TG-1. Escherichia coli TG-1 infected with phages displaying single-chain antibodies was spread on LB agar medium (ampicillin added to a final concentration of 50 mg/L) and incubated overnight at 37°C to allow colony formation. From these, 96 clones were randomly selected.

### (3) Experimental Procedure

1. A solution (PBS) containing 0.1 µg of AAV was applied to a Maxisorp 96-well plate, and allowed to stand at room temperature for 1 hour. As AAV samples, for AAV9, two types of samples containing full capsids or empty capsids, that is, an "AAV9 full capsid sample" and an "AAV9 empty capsid sample," were used.
2. The wells were washed twice with PBS.
3. 2% BSA blocking solution was applied to the plate and the plate was stirred under shaking at 25°C for 1 hour.
4. The wells were washed three times with PBST (0.1% Tween).
5. Separately, the 96 clones selected in the above (2) were each cultured in 2YT medium at 37°C for 24 hours, and the anti-AAV-scFv contained in the culture supernatant was purified using His MultiTrap FF according to the protocol and quantified. The 96 clones contained the first anti-AAV-scFv [1], the second anti-AAV-scFv [2], the third anti-AAV-scFv [3], and the fourth anti-AAV-scFv [4], which consist of the amino acid sequences of the variable regions shown in Table 2A, and the amino acid sequences of the linker and tag shown in Table 2B. Each of the anti-AAV-scFvs has a sequence configured, from the N-terminus, in the order of a heavy chain variable region, a linker, a light chain variable region, and a tag. An aqueous solution of each of the anti-AAV-scFvs [1] to [4] was applied to the plate, and stirred under shaking at 25°C for 1 hour (pH was approximately 7).

**[Table 2A]**

| | | FR1 | **CDR1** | FR2 | **CDR2** | FR3 | **CDR3** | FR4 | SEQ ID NO: |
|---|---|---|---|---|---|---|---|---|---|
| [1] | HV | QSLEESGGGLVKPGASLTLTCTAS | **GFSFSSYG** | ISWFRQAPGKGLEWIGT | **IYGGSSGST** | YYASWAKGRFTISKTSSTTMTLOMASLTAADTATYFC | **ARDRVGGNGYADL** | WGPGTLVTVTS | 21 |
| | LV | ELVMTQTAASVEAAVGGTVTIKCQAS | **QSISSY** | LAWYQQKPGQRPKLLIY | **RAS** | TLASGVSSRFKGSGSGTEYTLTISDLECDDAATYYC | **QSHYDIRNYGVA** | FGGGTEVDIK | 22 |
| [2] | HV | QSLEESGGDLVKPGASLTLTCTAS | **GFSFSSNYW** | MFW/ROAPGKGLEWIAH | **IYGGSSGST** | YYASWAKGRFTISKTSSTTVTLQMTSLTAADTATYFC | **ATVDNGYSDLKI** | WGPGTLVTVTS | 23 |
| | LV | ELVLTQTADSVEAGVGGTVTIKCQAS | **QSIRNW** | LSVVYQQKPGQFIPKLLIS | **RAS** | TLASGVPSRFSGSGSGTEFTLTISDLECDDGATYYC | **QSSFYADSGEPA** | FGGGTEVDIK | 24 |
| [3] | HV | QSVEESGGDLVKPGASLTLTCTAS | **GIDFSSGYD** | MCWvRQAPGKGLEWIGC | **IYTGGDSA** | WYASWAKGRFTISKTSSTTVTLQMTSLTGADTATYFC | **ANDLDSGTVSI** | WGPGTLVTVTS | 25 |
| | LV | ELVLTQTAASVSEPVGGTVTIKCQAS | **QSISSW** | LSNYQQKPGQPPKLLIY | **QAS** | KLASGVPSRFKGSGSGTQFTLTISDNCDDAATYYC | **AGWTSSTTDGTA** | FGGGTEVVI | 26 |
| [4] | HV | QSLEESGGDLVKPGASLTLTCTAS | **GFSFSSSAY** | MCVWRQAPGKGLEWIGC | **MHTISSVDT** | YYASWAKGRFAISKASSTTVTLQMTSL TMDTATYFC | **ARDDRNGYGYPYSFDI** | WGPGTLVTVTS | 27 |
| | LV | ELDLTQTAASVEAAVGGTITIKCQAS | **QTISSY** | LAWYQQKPGQPPKLLIY | **RAS** | TLASGVSSRFKGSGSGTEYTLTISDLECADAATYYC | **LTYYGNSHN** | FGGGTEVVIK | 28 |

| | | | | | | | | | |
|---|---|---|---|---|---|---|---|---|---|
| HV: heavy chain variable region; LV: light chain variable region | | | | | | | | | |

**[Table 2B]**

| | | | SEQ ID NO: |
|---|---|---|---|
| [1] | Linker | GGGGSGGGGSGGGGS | 57 |
| | Tag | RSAAAGGGSEQKLISEEDLGGGSHHHHHH* | 58 |
| [2] | Linker | GGGGSGGGGSGGGGS | 57 |
| | Tag | RSAAAGGGSEQKLISEEDLGGGSHHHHHH* | 58 |
| [3] | Linker | GGGGSGGGGSGGGGS | 57 |
| | Tag | TGSAAAGGGSEQKLISEEDLGGGSHHHH* | 59 |
| [4] | Linker | GGGGSGGGGSGGGGS | 57 |
| | Tag | GSAAAGGGSEQKLISEEDLGGGSHHHHH* | 60 |

6. The wells were washed three times with PBST (0.1% Tween).
7. An HRP-labeled anti-His-Tag antibody was applied to the plate and stirred under shaking at 25°C for 1 hour.
8. The wells were washed three times with PBST (0.1% Tween).
9. An HRP enzyme substrate (1-Step Ultra TMB-Substrate Solution) was added.
10. After incubation at room temperature to allow color development, sulfuric acid or hydrochloric acid was added to stop the reaction.
11. The absorbances at 450 nm and 650 nm were measured in each well, and the value obtained by subtracting the absorbance at 650 nm from the absorbance at 450 nm was defined as the ELISA signal absorbance.
12. The ratio of the absorbance for the full capsid sample (IF) to the absorbance for the empty capsid sample (IE), (IF/IE), and the ratio of the absorbance for the empty capsid sample (IE) to the absorbance for the blank (BSA) (IB), (IE/IB), were derived.

### Results

The ratios IF/IE and IE/IB obtained for the first anti-AAV-scFv [1], the second anti-AAV-scFv [2], the third anti-AAV-scFv [3], and the fourth anti-AAV-scFv [4] are shown in Table 3 below.

**[Table 3]**

| | IF/IE | IE/IB |
|---|---|---|
| [1] | 6.54 | 1.50 |
| [2] | 8.23 | 3.70 |
| [3] | 6.11 | 2.22 |
| [4] | 7.17 | 3.09 |

As shown in Table 3, all of the anti-AAV-scFvs [1] to [4] were found to have specific binding affinity for AAV full capsids.

### [Test Example 2]

Using the second anti-AAV-scFv [2], the same procedure as in Test Example 1 was performed, except that the amount of AAV applied to the plate in "1" of the experimental procedure of Test Example 1 was changed; the absorbances at 450 nm and 650 nm were measured in each well, and the value obtained by subtracting the absorbance at 650 nm from the absorbance at 450 nm was defined as the ELISA signal absorbance. FIG. 1 shows a graph with absorbance on the vertical axis and the amount of applied AAV on the horizontal axis.

As shown in FIG. 1, there was substantially no difference in absorbance between the blank (BSA) and the empty capsid (Empty), and no concentration dependence was observed, whereas the absorbance increased in a concentration-dependent manner for the full capsid (Full). That is, it was confirmed that AAV full capsids can be selectively quantified using the anti-AAV-scFv.

## Claims

1. An anti-AAV full capsid molecule that is an antibody having higher binding affinity for AAV full capsids than for AAV empty capsids.

2. An anti-AAV full capsid molecule comprising:
a heavy chain variable region including, as heavy chain complementarity-determining regions, CDR1 consisting of an amino acid sequence set forth in SEQ ID NO: 1, CDR2 consisting of an amino acid sequence set forth in SEQ ID NO: 2, and CDR3 consisting of an amino acid sequence having 90% or more sequence identity with an amino acid sequence set forth in SEQ ID NO: 3; and
a light chain variable region including, as light chain complementarity-determining regions, CDR1 consisting of an amino acid sequence set forth in SEQ ID NO: 4, CDR2 consisting of RAS, and CDR3 consisting of an amino acid sequence having 90% or more sequence identity with an amino acid sequence set forth in SEQ ID NO: 5.

3. An anti-AAV full capsid molecule comprising:
a heavy chain variable region including, as heavy chain complementarity-determining regions, CDR1 consisting of an amino acid sequence set forth in SEQ ID NO: 6, CDR2 consisting of an amino acid sequence set forth in SEQ ID NO: 7, and CDR3 consisting of an amino acid sequence having 90% or more sequence identity with an amino acid sequence set forth in SEQ ID NO: 8; and
a light chain variable region including, as light chain complementarity-determining regions, CDR1 consisting of an amino acid sequence set forth in SEQ ID NO: 9, CDR2 consisting of RAS, and CDR3 consisting of an amino acid sequence having 90% or more sequence identity with an amino acid sequence set forth in SEQ ID NO: 10.

4. An anti-AAV full capsid molecule comprising:
a heavy chain variable region including, as heavy chain complementarity-determining regions, CDR1 consisting of an amino acid sequence set forth in SEQ ID NO: 11, CDR2 consisting of an amino acid sequence set forth in SEQ ID NO: 12, and CDR3 consisting of an amino acid sequence having 90% or more sequence identity with an amino acid sequence set forth in SEQ ID NO: 13; and
a light chain variable region including, as light chain complementarity-determining regions, CDR1 consisting of an amino acid sequence set forth in SEQ ID NO: 14, CDR2 consisting of QAS, and CDR3 consisting of an amino acid sequence having 90% or more sequence identity with an amino acid sequence set forth in SEQ ID NO: 15.

5. An anti-AAV full capsid molecule comprising:
a heavy chain variable region including, as heavy chain complementarity-determining regions, CDR1 consisting of an amino acid sequence set forth in SEQ ID NO: 16, CDR2 consisting of an amino acid sequence set forth in SEQ ID NO: 17, and CDR3 consisting of an amino acid sequence having 90% or more sequence identity with an amino acid sequence set forth in SEQ ID NO: 18; and
a light chain variable region including, as light chain complementarity-determining regions, CDR1 consisting of an amino acid sequence set forth in SEQ ID NO: 19, CDR2 consisting of RAS, and CDR3 consisting of an amino acid sequence set forth in SEQ ID NO: 20.

6. The anti-AAV full capsid molecule according to any one of claims 2 to 5, wherein
the heavy chain variable region includes:
FR1 consisting of an amino acid sequence having 70% or more sequence identity with an amino acid sequence at positions 1 to 24 of SEQ ID NO: 21, positions 1 to 24 of SEQ ID NO: 23, positions 1 to 24 of SEQ ID NO: 25, or positions 1 to 24 of SEQ ID NO: 27;
FR2 consisting of an amino acid sequence having 70% or more sequence identity with an amino acid sequence at positions 33 to 49 of SEQ ID NO: 21, positions 34 to 50 of SEQ ID NO: 23, positions 34 to 50 of SEQ ID NO: 25, or positions 34 to 50 of SEQ ID NO: 27;
FR3 consisting of an amino acid sequence having 70% or more sequence identity with an amino acid sequence at positions 59 to 95 of SEQ ID NO: 21, positions 60 to 96 of SEQ ID NO: 23, positions 59 to 95 of SEQ ID NO: 25, or positions 60 to 96 of SEQ ID NO: 27; and
FR4 consisting of an amino acid sequence having 70% or more sequence identity with an amino acid sequence at positions 109 to 119 of SEQ ID NO: 21, positions 109 to 119 of SEQ ID NO: 23, positions 107 to 117 of SEQ ID NO: 25, or positions 113 to 123 of SEQ ID NO: 27; and
the light chain variable region includes:
FR1 consisting of an amino acid sequence having 70% or more sequence identity with an amino acid sequence at positions 1 to 26 of SEQ ID NO: 22, positions 1 to 26 of SEQ ID NO: 24, positions 1 to 26 of SEQ ID NO: 26, or positions 1 to 26 of SEQ ID NO: 28;
FR2 consisting of an amino acid sequence having 70% or more sequence identity with an amino acid sequence at positions 33 to 49 of SEQ ID NO: 22, positions 33 to 49 of SEQ ID NO: 24, positions 33 to 49 of SEQ ID NO: 26, or positions 33 to 49 of SEQ ID NO: 28;
FR3 consisting of an amino acid sequence having 70% or more sequence identity with an amino acid sequence at positions 53 to 88 of SEQ ID NO: 22, positions 53 to 88 of SEQ ID NO: 24, positions 53 to 88 of SEQ ID NO: 26, or positions 53 to 88 of SEQ ID NO: 28, and
FR4 consisting of an amino acid sequence having 70% or more sequence identity with an amino acid sequence at positions 101 to 110 of SEQ ID NO: 22, positions 101 to 110 of SEQ ID NO: 24, positions 101 to 109 of SEQ ID NO: 26, or positions 98 to 107 of SEQ ID NO: 28.

7. The anti-AAV full capsid molecule according to claim 2, wherein the heavy chain variable region includes an amino acid sequence set forth in SEQ ID NO: 21, and the light chain variable region includes an amino acid sequence set forth in SEQ ID NO: 22.

8. The anti-AAV full capsid molecule according to claim 3, wherein the heavy chain variable region includes an amino acid sequence set forth in SEQ ID NO: 23, and the light chain variable region includes an amino acid sequence set forth in SEQ ID NO: 24.

9. The anti-AAV full capsid molecule according to claim 4, wherein the heavy chain variable region includes an amino acid sequence set forth in SEQ ID NO: 25, and the light chain variable region includes an amino acid sequence set forth in SEQ ID NO: 26.

10. The anti-AAV full capsid molecule according to claim 5, wherein the heavy chain variable region includes an amino acid sequence set forth in SEQ ID NO: 27, and the light chain variable region includes an amino acid sequence set forth in SEQ ID NO: 28.

11. The anti-AAV full capsid molecule according to any one of claims 1 to 5, which is an IgG antibody, a Fab antibody, a Fab' antibody, a F(ab')² antibody, an Fv antibody, an scFv antibody, a dsFv antibody, an scFv-Fc antibody, a dsFv-Fc antibody, a Bis-scFv antibody, a minibody, a diabody, a triabody, or a tetrabody.

12. An affinity solid phase for binding AAV full capsids, the affinity solid phase comprising the anti-AAV full capsid molecule described in any one of claims 1 to 5, and a solid-phase material with the anti-AAV full capsid molecule immobilized thereon.

13. A method for capturing AAV full capsids, the method comprising a step 1 of bringing a sample containing AAV empty capsids and AAV full capsids into contact with the affinity solid phase for binding AAV full capsids described in claim 12, thereby capturing the AAV full capsids.

14. The method according to claim 13, further comprising a step 2 of quantifying the captured AAV full capsids by enzyme-linked immunosorbent assay.

15. A nucleic acid encoding the anti-AAV full capsid molecule described in any one of claims 1 to 5.

16. An expression cassette or a recombinant vector comprising the nucleic acid described in claim 15.

17. A transformant obtained by transforming a host using the expression cassette or recombinant vector described in claim 16.

18. A method for producing an anti-AAV full capsid molecule, the method comprising culturing the transformant described in claim 17.
